# EUROPÄISCHE PATENTANMELDUNG

(11) **EP 2 011 432 A1**
(43) Veröffentlichungstag der Anmeldung: **07.01.2009**
(21) Anmeldenummer: 08011922.5
(22) Anmeldetag: 02.07.2008
(51) Int. Cl.: A61B 1/303

(54) **Gynäkolgiesonde**

(30) Priorität: 04.07.2007 DE 202007009405 U
(71) Anmelder: Mylius, Harald, 84513 Töging (DE)
(72) Erfinder: Mylius, Harald, 84513 Töging (DE)
(74) Vertreter: Gössmann, Christoph Tassilo

(57) **Zusammenfassung**

Gynäkologiesonde, wobei es sich um eine im wesentlichen nicht gekrümmte gerade Sonde aus Glas handelt, die an einem Ende ein Metallstück, dass mit einer Energie liefernden Vorrichtung verbunden sein kann, aufweist, in ihrem Inneren einen länglichen Hohlkörper aufweist, der von der äußeren Sondenhülle umfasst wird, so dass sich ein Hohlraum zwischen dem inneren Hohlkorper und der äußeren Sondenhülle ausbildet, wobei der innere Hohlkörper ein elektrisch leitendes Gas enthält und sich bis zur Spitze erstreckt, um diese letztlich zu bilden und an ihrem oberen Ende gcächlossen ist und die Spitze als kurzer Stummel aus dem Grundkörper der Sonde senkrecht hervorragt.

## Beschreibung

Die Erfindung betrifft eine Gynäkologiesonde.

Bisher wurden in der Gynäkolgie Infektionen im vaginalen Bereich, Bereich der Gebärmutter mit Antibiotika behandelt, wie, insbesondere bei Vulvovaginitis und Salpingitis, Gonorrhoe, Syphilis etc.

Es ist Aufgabe der Erfindung den Stand der Technik zu verbessern, insbesondere vaginale Infektionen ohne Antibiotika zu behandeln, sowie Enzündungen im vaginalen Bereich zu behandeln.

Gynäkologiesonde, wobei sich um eine im wesentlichen nicht gekrümmte gerade Sonde aus Glas handelt, die an einem Ende ein Metallstück, dass mit einer Energie liefernden Vorrichtung verbunden sein kann, aufweist, in ihrem Inneren einen länglichen Hohlkörper aufweist, der von der äußeren Sondenhülle umfasst wird, so dass sich ein Hohlraum zwischen dem inneren Hohlkörper und der äußeren Sondenhüle ausbildet, wobei der innere Hohlkörper ein elektrisch leitendes Gas enthält und sich bis zur Spitze erstreckt, um diese letztlich zu bilden und an ihrem oberen Ende geschlossen ist und die Spitze als kurzer Stummel aus dem Grundkörper der Sonde senkrecht hervorragt, wobei das Verhältnis des Durchmessers der Spitze zu ihrer Länge mit der sie aus der Sonde heraus ragt vorzugsweise im wesentlichen 1 : 2 bis 1 : 3 beträgt.

Die erfindungsgemäße Gynäkologiesonde befindet sich in einem Handgriff eines Behandlungsgeräts.

Das Behandlungsgerät, weist eine Vorrichtung auf, die ein elektrisches oder elektromagnetisches Feld erzeugt, vorzugsweise mit einer Feldspannung von 1.800 V bis 35.000 V mittels einer Spannung von 12 V bis 600 V, einer Stromstärke von 0,1 µA bis 100 µA und einer Frequenz von 10.000 Hz bis 35.000 Hz.

Das elektrische oder elektromagnetische Feld wird vorzugsweise mittels einer Spannung von 12 V bis 600 V, bevorzugt einer Spannung von 12 V bis 50 V, besonders bevorzugt von 18 V bis 20 V, vorzugsweise einer Stromstärke von 0,1 µA bis 100 µA, bevorzugt einer Stromstärke von 0,1 µA bis 20 µA, besonders bevorzugt mit einer Stromstärke von 0,8 µA bis 10 µA und vorzugsweise einer Frequenz von 10.000 bis 50.000 Hz, bevorzugt mit einer Frequenz von 25.000 Hz bis 40.000 Hz, besonders bevorzugt mit einer Frequenz von 25.000 Hz bis 38.000 Hz, erzeugt. Die Spannung im elektrischen oder elektromagnetischen Feld beträgt vorzugsweise 1.800 bis 35.000 V, bevorzugt 8.000 bis 18.000 V und besonders bevorzugt 12.000 bis 18.000 V beträgt.

Die erfindungsgemäße Gynäkologiesonde mit dem Behandlungsgerät ist vorzugsweise zur Anwendung bei Menschen und Tieren gedacht.

Das erfindungsgemäße Behandlungsgerät weist vorzugsweise in einem Handgriff einen Hochspannungstrafo mit Kammerwicklung in Form von seriell geschalteten Spulen, vorzugsweise 12 bis 20, bevorzugt 14 bis 18, besonders bevorzugt 16 auf, die vorzugsweise einen speziellen Kern, vorzugsweise einen Stabkern mit einer Anfangspermeabilität µi von vorzugsweise 350 bis 850, bevorzugt 450 bis 750, besonders bevorzugt 550 bis 650, ganz besonders bevorzugt 600 aufweisen, um eine Feldspannung von 1.800 V bis 35.000 V zu erzeugen. Die oben erwähnte kontrollierte Frequenz von 10.000 Hz bis 50.000 Hz wird mit einer Wiederholungsfrequenz von vorzugsweise 350 Hz bis 500 Hz, bevorzugt 400 Hz bis 480 Hz, besonders bevorzugt 430 Hz bis 460 Hz, ganz besonders bevorzugt 450 Hz oder vorzugsweise 1080 Hz bis 1280 Hz Hz (für die schmerzfreie Behandlung), bevorzugt 1120 bis 1240 Hz (für die schmerzfreie Behandlung) aufgebaut. Diese Pulsfrequenz wird über einen Halbleiterschalter, vorzugsweise einen MOS Schalttransistor erzielt, der vorzugsweise im Handgriff plaziert ist, um eine bessere Leistung ohne Induktivität und kapazitive Verluste des Zuführkabels zu erzielen. Diese Pulsart ist ein kurzer Rechtecksimpuls, der eine gedämpfte Schwingung anregt. Die Pulsbreite ist zwischen 1 und 30 µs, bevorzugt 1 bis 13 µs einstellbar, was die Leistung der Erzeugung des atomaren Sauerstoffs von 5% bis 100% bei der höchsten Leistungsstufe steuert.

Der kleine in Kammern gewickelte Hochspannungstrafo ist im Handgriff geschirmt und erzeugt nur minimale elektromagnetische Störungen. Der Gehäuseableitstrom ist dabei mit kleiner 10 µA bei voller Leistung sehr gering. Bei einem Patientenhilfsstrom von größer als 120 µA schaltet die Elektronik vorzugsweise automatisch ab. Die Heilungszeit, die Patientenhilfsstromüberwachung und Abschaltung wird vorzugsweise über einen Mikrochip geregelt. In klinischen Versuchen wurde eine Heilungszeit von vorzugsweise 30 - 60 sec bei der Behandlung ermittelt. Dies kann vorzugsweise über einen Signalton angegeben werden, z.B. alle 10 sec.

An dem Handgriff befindet sich eine Sonde, vorzugsweise eine hohle Glassonde, die mit einem elektrisch leitenden Gas, also einem elektronisch anregbarem ionisierbaren Gas gefullt ist, vorzugsweise mit zumindest einem Edelgas oder beliebigen Edelgasgemischen gefüllt ist.

Bei der erfindungsgemäßen Gynäkologiesonde handelt es sich um eine im wesentlichen vorzugsweise nicht gekrümmte gerade, rund Sonde aus Glas, die an einem Ende ein Metallstück, dass mit einer Energie liefernden Vorrichtung verbunden sein kann, aufweist, in ihrem Inneren einen langlichen Hohlkörper aufweist, der von der äußeren Sondenhülle umfasst wird, so dass sich ein Hohlraum zwischen dem inneren Hohlkörper und der äußeren Sondenhülle ausbildet, wobei der innere Hohlkörper ein elektrisch leitendes Gas enthält und sich bis zur Spitze erstreckt, um die Spitze läuft vorzugweise eine Kavitat in Form einer Vertiefung, die eine Rille bildet und das Verhaltnis des Durchmessers der Spitze zu ihrer Länge mit der sie aus der Sonde heraus ragt, vorzugsweise im wesentlichen 1 : 2 bis 1 : 3 und das Verhältnis der Länge der Spitze zum Grundkörper der Sonde 1 : 38 bis 1 : 15 beträgt.

In einer bevorzugten Ausführungsform besteht die Gynäkologiesonde aus einem Hohlraum aus Glas, der evakuiert oder nicht evakuiert sein kann, in dem sich vorzugsweise ein weiterer Hohlraum aus Glas befindet, der mit Gas gefüllt ist und sich bis zum oberen Ende der Gynäkologiesonde erstreckt, also in die Spitze der Gynäkologiesonde übergeht. Die Glassonde weist an ihrem unteren Ende, mit dem sie in dem Handgriff vorzugsweise über eine formschlüssige Klemmverbindung befestigt ist, an einem Ende ein Metallstück auf, dass mit einer Energie liefernden Vorrichtung verbunden sein kann, bevorzugt einen zylinderförmigen Metallverschluss auf. Die Energie lieternde Vorrichtung ist eine derartige, die elektrischen Strom zur Verfügung stellt. Die Gynäkologiesonde weist eine Gesamtlänge von der Sondespitze bis zum Ende von 300 mm bis 150 mm bevorzugt von 250 mm bis 200 mm und besonders bevorzugt von 200 bis 150 mm und einen Durchmesser von 30 mm bis 10 mm, bevorzugt 25 bis 20 mm besonders bevorzugt von 20 mm bis 10 mm auf, die Größenangaben beziehen sich vorzugsweise auf den Einsatz bei Menschen, kann also in der Tiermedizin entsprechend nach unten oder oben abweichen.

Die Gynäkologiesonde, ist in einer bevorzugten Ausführungsform derart ausgebildet, dass das Endteil der Sonde vorzugsweise einen geringeren Durchmesser aufweist, als in ihrem mittleren oder oberen Teil.

Die Glassonde ist vorzugsweise mit zumindest einem leitenden Gas, vorzugsweise einem Edelgas, bei einem Unterdruck von vorzugsweise 0,1 bis 1000 mbar bevorzugt 0,1 bis 500 mbar, besonders bevorzugt 0,1 bis 10 mbar, ganz besonders bevorzugt 2 mbar bis 5 mbar gefüllt. Als Edelgase werden vorzugsweise beliebige Mischungen aus Edelgasen, vorzugsweise beliebige Mischungen aus Argon und Neon verwendet, wobei Mischungen, die mehr Neon als Argon enthalten, bevorzugt sind, vorzugsweise 0 Vol. % Argon bis 100 Vol. % Neon, bevorzugt 10 Vol.% Argon bis 90 Vol. % Neon, besonders bevorzugt 30 Vol. % Argon bis 70 % Neon, besonders bevorzugt ist eine Mischung aus 30 Vol.% Argon und 70 Vol.% Neon.

In einer weiteren bevorzugten Ausführungsform weist die Gynäkologiesonde eine stromableitende Vorrichtung zur Erdung auf, diese ist vorzugsweise in der Nähe der Stelle an der Sonde befestigt, die mit dem Körper in Kontakt oder nahe in Kontakt kommt. Bei dieser stromableitenden Vorrichtung handelt es sich vorzugsweise um eine beliebige Vorrichtung, die in der Lage ist Strom zu leiten, dies kann eine Flüssigkeit, ein Gas oder vorzugsweise eine Vorrichtung aus Metall sein, wie bevorzugt ein Draht aus Metall, der vom Glasende der Sonde an ihr entlang zur Masse geführt wird. Vorzugsweise wird der Draht Außen oder auch im Inneren der Sonde entlang zur Masse geführt. Falls der Draht im Inneren der Sonde geführt wird, muss die Sonde doppelwandig sein. Die stromableitende Vorrichtung, wie zum Beispiel eine stromleitende Flüssigkeit oder stromleitendes Gas, vorzugsweise ein Draht aus Metall, kann sich auch vorzugsweise in einem an der Gynäkologiesonde befestigtem Rohr aus vorzugsweise Kunststoff, Metall oder bevorzugt Glas befinden. Der Draht ist vorzugsweise in der Nähe der Stelle an der Sonde befestigt, die mit dem Körper in Kontakt oder nahe in Kontakt kommt, wobei zwischen der Ableitung, also dem Draht und der Glassonde ein Luftraum ist, vorzugsweise wird der Draht am oberen Ende der Gynäkologiesonde befestigt, das mit dem Körper in Kontakt kommt, wobei bei einem konkaven Sondenende der Draht auch vorzugsweise in der Kavität befestigt sein kann, bei einem spitzen Sondenende vorzugsweise irgendwo entlang der Sondenspitze befestigt sein kann. Die Befestigung der stromableitenden Vorrichtung erfolgt vorzugsweise, indem der Draht am oberen Ende der Gynäkologiesonde in das Glas eingeschmolzen ist, angeklebt ist oder vorzugsweise über eine Klemmverbindung befestigt ist, die über die Spitze der Gynäkologiesonde gezogen wird. Diese Klemmverbindung kann als Ring ausgebildet sein, der aus Metall oder vorzugsweise einem Kunststoff ausgebildet ist, der vorzugsweise starr oder bevorzugt flexibel sein kann und bevorzugt ein Siliconelastomer ist. In einer bevorzugten Ausführungsform befindet sich die stromableitenden Vorrichtung in einer Aufnahmevorrichtung, die aus vorzugsweise einem elastischen Material, wie einem Metall oder bevorzugt einem Kunststoff aufgebaut ist, wobei die Aufnahmevorrichtung längs mittels einer Klemmverbindung an dem runden Sondenglaskörper befestigt und abgewinkelt ist, vorzugsweise mit einem Winkel 90° zu dem runden Sondenglaskörper und mit einer weiteren Klemmverbindung kreisförmig um den runden Sondenkopf greift, wobei vorzugsweise ein Draht aus Metall an der Aufnahmevorrichtung längs bis in den Kopf der Aufnahmevorrichtung geführt wird, in der er in der Mitte der kreisförmigen Öffnung endet, wobei der Draht sich sowohl im Abstand von dem Glaskopf der sonde als auch von der Öffnung der Aufnahmevorrichtung befindet. Überraschender Weise wurde festgestellt, dass nun auch Patienten, die besonders schmerzempfindlich sind, bei Gynäkologiesonden, die die stromableitende Vorrichtung zur Erdung aufweisen, nun überhaupt keinen Schmerz mehr empfinden.

Die Quelle für das oxidierende Gas ist derart aufgebaut, dass das Behandlungsgerät das oxidierende Gas direkt an einer Glassonde erzeugt, vorzugsweise aus dem umgebenden Luftsauerstoff oder auch aus reinem Sauerstoff, wobei sowohl der Luftsauerstoff als auch reiner Sauerstoff an den Applikationsort zusätzlich, vorzugsweise über einen schlauch, zugeführt werden kann, wobei die Gynäkologiesonde an dem Handgriff angebracht ist, indem nach dem Prinzip der stillen elektrischen Gasentladung ein elektrisches Feld zwischen zwei Polen, hier die Sonde und ein Patient, erzeugt wird, die durch einen Isolator, nämlich Luft getrennt ist. Ab einer Grenzfeldstärke werden in dem elektrischen Feld durch andauernde Entladevorgänge Elektronen erzeugt, die den in der Umgebungsluft enthaltenen Sauerstoff in ionisierten Sauerstoff, in Radikale spaltet, wie hochreaktive substanzen (Sauerstoff imstatu nascendi) wie atomarer Sauerstoff, der hauptsächlich gebildet wird, Hydroxylionen, Ozon und andere gebildet. Nach dem Anlegen einer Wechselspannung mit bis zu 35 000 Hz entsteht zwischen der Elektrode, der Glassonde und dem Patienten ein Entladevorgang und - hiermit verknüpft - ein elektrisches Feld mit hoher Elektronendichte. Die Radikalen entstehen, indem Moleküle der Luft von Elektronen getroffen werden und hierbei ein erhöhtes Energieniveau erreichen. Der atomare Sauerstoff, der hochreaktiv ist, reagiert mit den Keimen der Applikationsstelle, dabei entsteht auch etwas Ozon, das jedoch weit unter der MAK (maximale Arbeitsplatzkonzentration) Grenze von 0,1 ppm liegt, nämlich nur bei 18 % des MAK Werts. Überraschend ist, dass gerade im erfindungsgemäßen Bereich der Stromstärke und der Frequenz besonders viel atomarer Sauerstoff erzeugt wird. Der atomare Sauerstoff wird sowohl vor der Ozonbildung als auch beim Zerfall des Ozons gebildet. Das beim erfindungsgemäßen Behandlungsgerät gebildete Ozon zerfällt, soweit es vom Gewebe resorbiert wird, im wässerigen Milieu, insbesondere bei der Vaginalbehandlung, das heißt in der interzellulären Substanz innerhalb von ca. 10 Sekunden auf den halben Wert seiner ursprünglichen Konzentration.

Das Behandlungsgerät mit der erfindungsgemäßen Gynäkologiesonde ist auf Grund seiner niedrigen Stromstärke und hohen Bildung an atomaren Sauerstoff besonders geeignet zur Behandlung von Tieren und Menschen an der Hautoberfläche, um Keime zu beseitigen, die Durchblutung zu fördern und damit auch den Lymphabfluß zu erhöhen. Somit ist es besonders geeignet zur Vaginalbehandlung.

Das Behandlungsgerät mit der erfindungsgemäßen Gynäkologiesonde tötet alle Keime an der Applikationsstelle innerhalb von ca. 40 Sekunden.
**Figur 1**

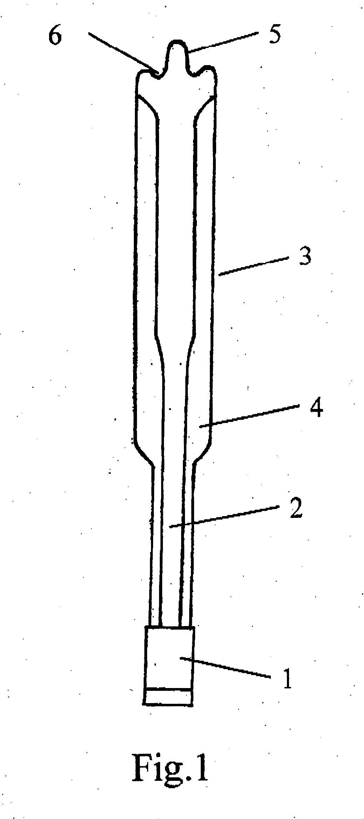

   Figur 1 zeigt die erfindungsgemäße Gynäkologiesonde vorzugsweise für den Portio (Muttermund), die an einem Ende ein Metallstück (1) aufweist, in ihrem Inneren einen länglichen Hohlkörper aufweist (2), der von der außeren Sondenhülle (3) umfasst wird, so dass sich ein Hohlraum (4) zwischen dem inneren Hohlkörper (2) und der äußeren Sondenhülle (3) ausbildet, wobei der innere Hohlkörper ein elektrisch leitendes Gas enthält und sich bis zur Spitze (5) erstreckt, um diese letztlich zu bilden und an ihrem oberen Ende geschlossen ist, wobei um die Spitze eine Kavität in Form einer Rille (6) ausgebildet ist und das Verhältnis des Durchmessers der Spitze zu ihrer Länge mit der sie aus der Sonde heraus ragt im wesentlichen vorzugsweise 1 :2 bis 1 : 3 beträgt.

## Patentansprüche

1. Gynäkologiesonde, **dadurch gekennzeichnet, dass** es sich um eine im wesentlichen nicht gekrümmte gerade Sonde aus Glas handelt, die an einem Ende ein Metallstück, dass mit einer Energie liefernden Vorrichtung verbunden sein kann, aufweist, in ihrem Inneren einen länglichen Hohlkörper aufweist, der von der äußeren Sondenhülle umfasst wird, so dass sich ein Hohlraum zwischen dem inneren Hohlkörper und der äußeren Sondenhülle ausbildet, wobei der innere Hohlkörper ein elektrisch leitendes Gas enthält und sich bis zur Spitze erstreckt, um diese letztlich zu bilden und an ihrem oberen Ende geschlossen ist und die Spitze als kurzer Stummel aus dem Grundkörper der Sonde senkrecht hervorragt.

2. Gynäkologiesonde nach Anspruch 1, **dadurch gekennzeichnet, dass** die Sonde eine Gesamtlänge von der Sondespitze bis zum Ende von 300 mm bis 150 mm und einen Durchmesser von 10 mm bis 30 mm aufweist.

3. Gynäkologiesonde, nach Anspruch 1 oder 2, **dadurch gekennzeichnet, dass** das Endteil der Sonde einen geringeren Durchmesser aufweist, als in ihrem mittleren Teil.

4. Gynäkologiesonde nach einem oder mehreren der Ansprüche 1 bis 3, **dadurch gekennzeichnet, dass** die Sonde mit zumindest einem Edelgas oder einem beliebigen Edelgasgemisch gefüllt ist.

5. Gynäkologiesonde nach einem oder mehreren der Ansprüche 1 bis 4, **dadurch gekennzeichnet, dass** die Sonde mit einem Edelgasgemisch aus Argon und Neon gefüllt ist.

6. Gynäkologiesonde nach einem oder mehreren der Ansprüche 1 bis 5, **dadurch gekennzeichnet, dass** die Sonde mit einem Edelgasgemisch aus 30 Vol.% Argon und 70 Vol.% Neon gefüllt ist.

7. Gynäkologiesonde nach einem oder mehreren der Ansprüche 1 bis 6, **dadurch gekennzeichnet, dass** die Behandlungssonde eine stromableitende Vorrichtung zur Erdung aufweist.

8. Gynäkologiesonde nach einem oder mehreren der Ansprüche 1 bis 7, **dadurch gekennzeichnet, dass** die Behandlungssonde über eine Vorrichtung betrieben wird, die ein elektrisches oder elektromagnetisches Feld mit einer Feldspannung von 1.800 bis 35.000 V mittels einer Spannung von 12 bis 600 V, einer Stromstärke von 0,1 µA bis 100 µA und einer Frequenz von 10.000 bis 35.000 Hz erzeugt.
